# EUROPEAN PATENT SPECIFICATION

(11) **EP 4 241 739 B1**
(45) Date of publication and mention of the grant of the patent: **10.07.2024**
(21) Application number: 23160570.0
(22) Date of filing: 07.03.2023
(51) Int. Cl.: A61F 2/30, A61F 2/38, A61F 2/46

(54) **HYBRID KNEE PROSTHESIS**
HYBRIDKNIEPROTHESE
PROTHÈSE DE GENOU HYBRIDE

(30) Priority: 07.03.2022 US 202263317123 P
(43) Date of publication of application: 13.09.2023
(73) Proprietor: Howmedica Osteonics Corporation, Mahwah, New Jersey 07430 (US)
(72) Inventor: KAYAL, Jordan, MAHWAH, NEW JERSEY 07430 (US); PATTNAIK, Pramit, 767033 BALANGIR ODISHA (IN); PASCALE, Kenneth, HOBOKEN, NEW JERSEY 07030 (US); DUNBAR, Michael James, DUNCANS COVE B3V 1K4 (CA)
(74) Representative: Regimbeau

(56) References cited:
- US-A1- 2015 164 645
- US-A1- 2019 070 008
- US-A1- 2019 358 044
- US-A1- 2021 228 366

## Description

### BACKGROUND OF THE INVENTION

Total knee arthroplasty ("TKA") is a common orthopedic procedure for knee joints. Prior to implanting artificial joint components into a knee joint of a patient, a surgeon generally resects at least a portion of the patient's native bone in order to create surfaces and/or recesses for accepting or receiving a least a portion of the prosthetic components. Generally, a surgeon only resects the amount of bone that is needed in order to properly implant the prosthetic components in the joint because once native bone is resected from a joint, it is gone forever. This is typically done using cutting jigs to perform multiple resections of a distal femur and a proximal tibia.

When previously implanted prosthetic components fail for any one of a variety of reasons, a revision procedure is often necessary. An issue generally encountered by surgeons replacing joints during a revision procedure is the additional loss of native bone near the joint being replaced. This bone loss is typically due to movement of the component after implantation, bone degeneration, and/or as incidental to the removal of the previous implant. In this regard, one or more void filling prosthesis may be used to support the remaining bone and the revision prosthesis. Additionally, the revision joint prosthesis, whether it be a femoral or tibial prosthesis, typically includes an intramedullary stem that is either press-fit in the diaphysis of the bone or secured using bone cement to provide further support of the artificial joint surfaces. In either fixation circumstance, an intramedullary stem brings a significant degree of complexity to the procedure as it may require special instrumentation to address variables that may only present themselves during the procedure, such as stem length and stem offset. Such complexity may add to the length of the surgical procedure, increase the chances of complications, and limit the scope of surgeons that have the necessary skill and/or experience to perform such procedures.

Intramedullary stems often extend through the metaphysis of the bone and into the diaphysis, which is generally narrow and constraining. Such constraint may limit the alignment methodology utilized by the surgeon to an anatomic alignment precluding other alignment methodologies, such as kinematic alignment. An anatomic alignment technique generally seeks to achieve a neutral joint line that is oriented perpendicular to a mechanical axis of the tibia. A kinematic alignment technique on the other hand seeks to return the knee to its original undegenerated joint line, whether it be naturally varus, valgus, or neutral.

Revision TKA prostheses are also typically designed as fixed composite beam constructs such that that the prosthesis stem is rigidly fixed in the bone. In this regard, the stem and bone adjacent to it may receive the majority of loading so that there is little to no controlled loading of the bone at the proximal tibial or distal femur by the joint component. This may lead to the deterioration of bone adjacent to the joint component due to stress-shielding. This can further result in prosthesis loosening due at least in part to internal-external rotational forces applied through the joint component to the stem which typically has a rounded cross-section that is poorly equipped to resist rotation. Therefore, further improvements are desirable. The closest prior art is document US 2015/164645 A1, which defines the preamble of claim 1.

### SUMMARY OF THE DISCLOSURE

The invention is defined in claim 1. In one aspect of the present disclosure, a total knee arthroplasty system includes a joint prosthesis that has a joint component and a stem extending from the joint component. The joint component has an articular side and a bone contact side. The articular side has first and second condyles. The bone contact side having a porous bone contact surface. The system also includes a void filling prosthesis that has a porous outer surface and a channel that extends entirely through the void filling prosthesis and is configured to receive the stem of the joint prosthesis. Also included in the system is a cement plug and a cement shield. The cement shield has a first member and a second member. The first member and the second member define a channel extending through the cement shield and have a planar surface configured to abut a resected surface of a bone so that the channel aligns with an intramedullary canal of the bone.

Additionally, the first and second members of the shield may be moveable relative to each other from an open position to a closed position. Each of the first and second members may include a shield body that includes a notch such that when the first and second members are in the closed position, the notches of the first and second members may come together to form the channel. The first and second members may be pivotably connected to each other. The first and second members may include a ratchet mechanism that secures the first and second members while in the open and closed positions.

The system may also include a cement pressurizer that has an elongate shaft and a flange that extends radially outwardly from an end of the elongate shaft. The flange may have a periphery corresponding to a periphery of the channel of the void filling prosthesis. The cement pressurizer may include a projection that extends from the flange in an axial direction and that has a depression therein. The projection may be cylindrical.

The joint prosthesis may be a tibial prosthesis, and the joint component may be a tibial baseplate. The void filling prosthesis may include notches that extend through a sidewall thereof and may be configured to receive keels of the tibial baseplate. The stem may include a connection portion and a stem portion. The stem portion may include a plurality of flutes and grooves forming a cruciform cross-sectional shape. Each flute may be tapered in a proximodistal direction and a direction transverse to the proximodistal direction.

The joint prosthesis may also be a femoral prosthesis, and the joint component may be a distal femoral component. The void filling prosthesis may have a frustoconical shape and may include a central body and a pair of opposing legs that define a space therebetween.

### BRIEF DESCRIPTION OF THE DRAWINGS

The features, aspects, and advantages of the present invention will become better understood with regard to the following description, appended claims, and accompanying drawings in which:
FIG. 1A is a front view of a tibial baseplate according to an embodiment of the present disclosure.
FIG. 1B is a bottom view of the tibial baseplate of FIG 1A.
FIG. 2A is a perspective view of a stem according to an embodiment of the present disclosure.
FIG. 2B is a side view of the stem of FIG. 2A.
FIG. 2C is a cross-sectional view of the stem taken along line C-C of FIG. 2B.
FIG. 3 is a perspective view of a void filling prosthesis according to an embodiment of the present disclosure.
FIG. 4 is a perspective view of a cement plug according to an embodiment of the present disclosure.
FIG. 5 is perspective view of a cement restrictor tool according to an embodiment of the present disclosure.
FIG. 6A is a perspective view of cement pressurizer according to an embodiment of the present disclosure.
FIG. 6B is a side view of the cement pressurizer of FIG. 6A.
FIG. 6C is a bottom view of the cement pressurizer of FIG. 6A.
FIGS. 7A and 7B are schematics illustrating a method that may be followed using the present invention.
FIG. 8A is a front view of a tibial baseplate according to another embodiment of the present disclosure.
FIG. 8B is a perspective view of a stem of the tibial baseplate of FIG. 8A according to another embodiment of the present disclosure.
FIGS. 9A and 9B are schematics illustrating a method that may be followed using the present invention.
FIG. 10A is a top view of a cement restrictor tool according to another embodiment of the present disclosure.
FIG. 10B is a front view of the cement restrictor tool of FIG. 10A.
FIG. 11A is perspective view of a cement pressurizer according to another embodiment of the present disclosure.
FIG. 11B is a bottom view of the cement pressurizer of FIG. 11A.
FIG. 12A is a side view of a femoral prosthesis assembly according to an embodiment of the present disclosure.
FIG. 12B is a perspective view of a void filling prosthesis of the assembly of FIG. 12A according to another embodiment of the present disclosure.
FIG. 13 is a schematic illustrating a method that may be followed using the present invention.

### DETAILED DESCRIPTION

When referring to specific directions in the following discussion of certain implantable devices, it should be understood that such directions are described with regard to the implantable device's orientation and position during exemplary application to the human body. Thus, as used herein, the term "proximal" means close to the heart and the term "distal" means more distant from the heart. The term "anterior" means toward the front of the body or the face, and the term "posterior" means toward the back of the body. The term "medial" means toward the midline of the body, and the term "lateral" means away from the midline of the body. Also, as used herein, the terms "about," "generally" and "substantially" are intended to mean that slight deviations from absolute are included within the scope of the term so modified.

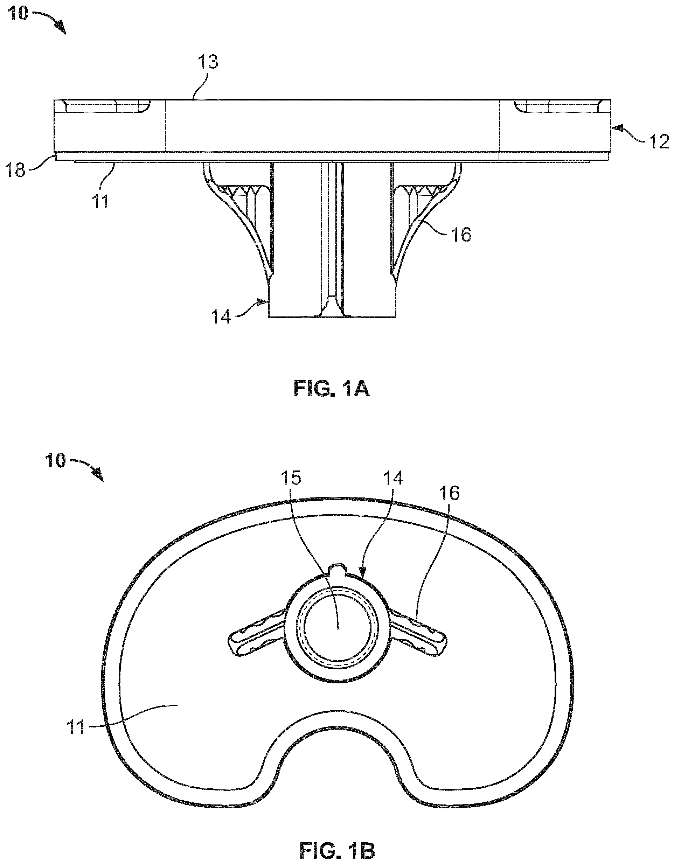

FIGs. 1A and 1B depict a modular tibial baseplate 10 according to an embodiment of the present disclosure. Tibial baseplate 10 generally includes a plate portion 12, stem boss 14, and keels 16. Plate portion 12 includes an articular side and a bone contact side. The articular side defines a tray 13 that is configured to receive a tibial insert (not shown) which has lateral and medial condylar surfaces for articulation with a femoral component. The bone contact side includes a layer of porous material 18 configured to promote bony ingrowth. An exemplary porous material is disclosed in U.S. Patent No. 10,716,673. Such layer of porous material 18 defines a generally planar bone contact surface 11. Stem boss 14 extends from the bone contact side of plate portion 12 and has a connection opening 15 configured to connect to a stem. In this regard, opening 15 may be tapered for a taper-lock connection or internally threaded for a threaded connection, for example. Keels 16 extend from the bone contact side of plate portion 12 and from lateral and medial sides of stem boss 14. Keels 16 help provide rotational resistance when implanted in a tibia.

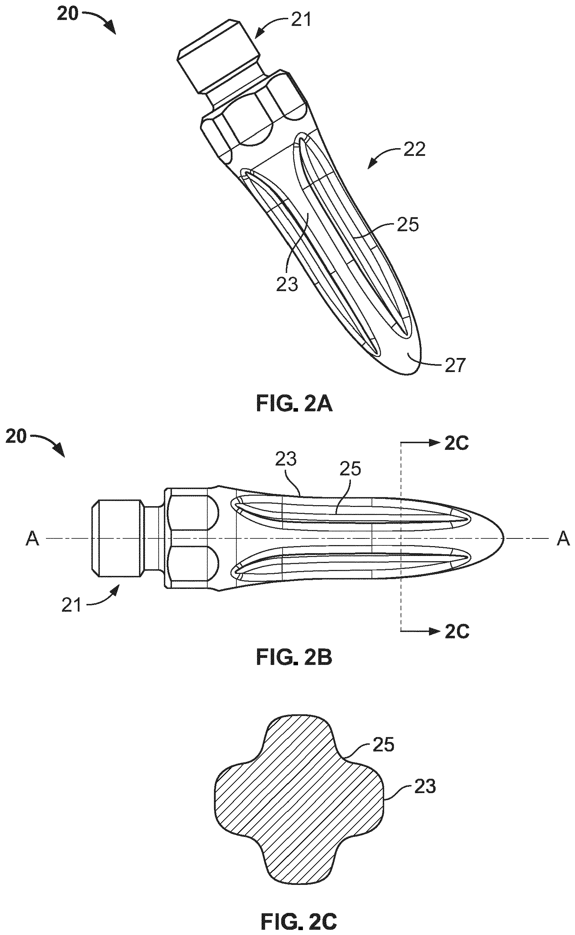

FIGs. 2A-2C depict a modular stem 20 according to an embodiment of the present disclosure. Stem 20 generally includes a connection portion 21 and stem portion 22. Connection portion 21 is configured to connect to stem boss 14 of baseplate 12. In this regard, connection portion 21 may be tapered for a taper-lock connection or threaded for a threaded connection. Stem portion 22 extends from connection portion 21 and includes a plurality of flutes 23 and grooves 25 that extend along a length of stem portion 22 to a rounded tip thereof 27. In the embodiment depicted, stem portion 22 includes four flutes 23 and four grooves 25 so that stem portion 22 forms a cruciform cross-sectional shape, as best shown in FIG. 2C. However, more or less flutes 23 may be provided to form alternative cross-sectional shapes, such as a star shape, for example. Flutes 23 are tapered in the proximodistal, mediolateral, and anteroposterior directions. In other words, each flute 23 is tapered in the proximodistal direction and a direction transverse to the proximodistal direction. Grooves 25 have a uniform depth along their length for uniform loading and enhanced cement-stem interaction. The cruciform cross-section allows bone cement to become interposed within grooves 25 while flutes 23 resist rotation relative to the bone cement. Additionally, the multidirectional taper of flutes 23 facilitates subsidence of stem 20 within bone cement, as discussed in more detail below.

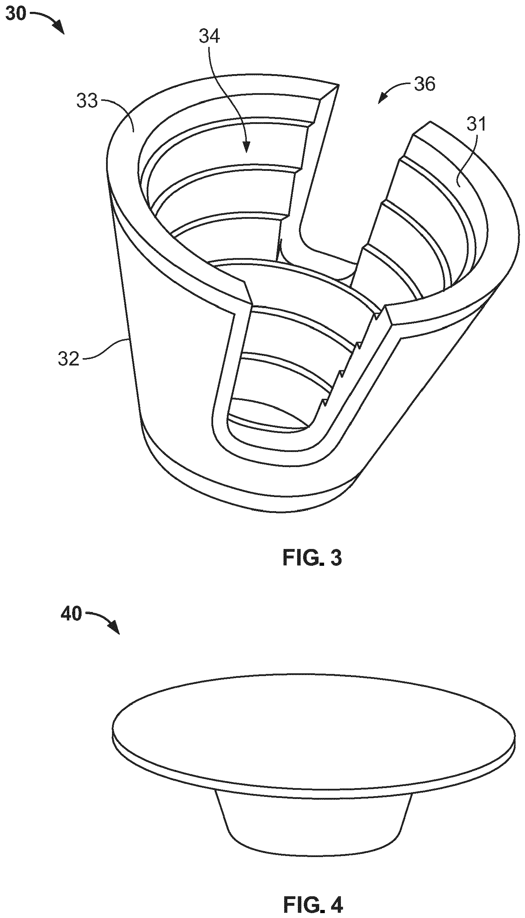

FIG. 3 depicts a tibial void filling prosthesis 30 according to an embodiment of the present disclosure. Void filling prosthesis 30 has a frustoconical shape such that its periphery extends about a longitudinal axis and tapers along the longitudinal axis from a first end to a second end thereof. A channel 34 extends entirely through prosthesis 30 and is defined by an interior surface 31. Interior surface 31 may be roughened or corrugated, as shown, to help prevent migration of bone cement disposed within channel 34. A layer of porous material 32 forms an exterior surface of prosthesis 30 to promote bony ingrowth. Notches 36 extend through a sidewall 33 of prosthesis 30 from the first end thereof which forms a clearance space configured to receive keels 16. However, in some embodiments, prosthesis 30 may not have such notches 36. Void filling prosthesis 30 can have other shapes and configurations such as those disclosed in U.S. Patent Nos. 9,011,444; 9,149,282; 10,524,806 and 10,299,929.

FIG. 4 depicts a cement plug or restrictor 40. Plug 40 is made of a generally pliable biocompatible material and is configured to secure itself within an intramedullary canal of a bone to restrict migration of bone cement.

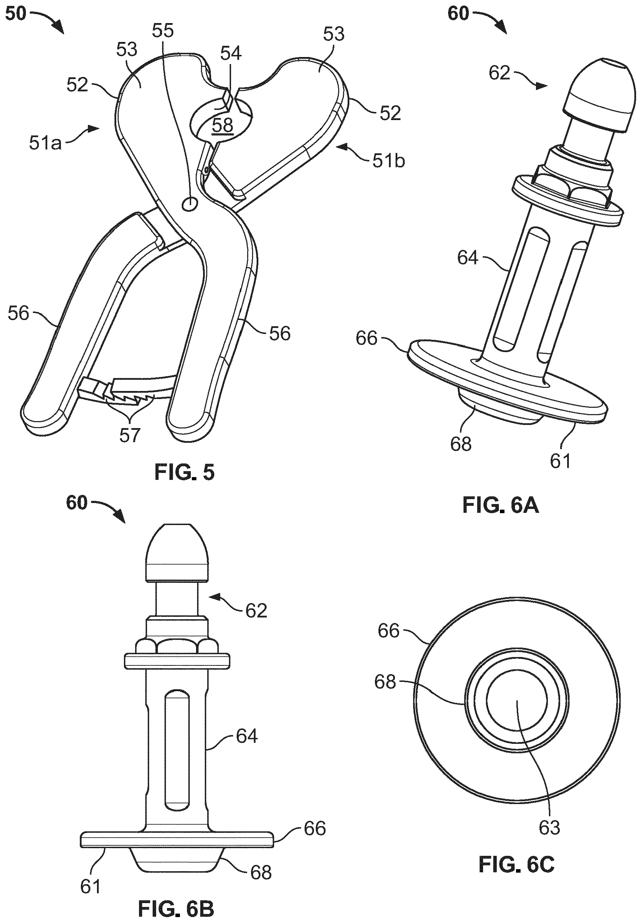

FIG. 5 depicts a cement shield 50 according to an embodiment of the present disclosure. Cement shield 50 generally includes a first member 51a and a second member 51b which each include a plate or shield body 52 and a handle 56 extending from shield body 52. First and second members 51a-b are pivotably connected via a pin 55 at a location between handles 56 and shield bodies 52. This allows shield bodies 52 to be moved closer to or further away from each other by actuation of handles 56. Each shield body 52 is shaped to match a portion of a resected end of a bone. In particular, each shield body 52 is shaped to match a portion of a resected proximal tibia which ordinarily has a planar resected surface. In this regard, each shield body 52 has a planar shield surface 53 for engagement with the resected surface and, when shield bodies 52 are brought together, they collectively form a shape that can cover the proximal resected surface of a tibia. In an embodiment configured for a resected femur (not shown), each shield body may have two to five planar intersecting surfaces that each correspond to a resected surface of a distal femur for conformal engagement therewith.

Each shield body 52 defines a notch 54 that faces the notch 54 of the other shield body 52 so that when the shield bodies 52 are brought together, notches 54 form a channel 58, which is shown to be circular to correspond to a periphery of stem 20 but with a larger diameter than a maximum major diameter of stem 20. Each handle has a ratchet arm 57 extending therefrom that engages the ratchet arm 57 of the other handle 56 to form a ratchet mechanism. The ratchet mechanism helps secure first and second members 51a-b in one of a plurality of positions. For example, first and second members 57 may have a first or closed position in which shield bodies 52 either touch or are nearly touching, and a second or open position in which shield bodies 52 are separated from each other by a distance greater than in the first position. In the closed position, stem 20 can be inserted through channel 58. In the open position, channel 58 is opened so that shield bodies 51a-b are able to be maneuvered away from stem 20.

FIGs. 6A-6B depict a cement pressurizer 60 according to an embodiment of the present disclosure. Pressurizer 60 generally includes a connection portion 62, shaft 64, flange 66, and projection 68. Connection portion 62 is configured to connect, via a quick-connect mechanism or otherwise, to another instrument, such as an impaction handle for impaction by a mallet or the like. Shaft 64 extends from connection portion 62. Flange or plate is disposed at an end of shaft 64 opposite that of connection portion 62 and has a planar distal surface 61. Flange extends 66 radially outwardly and has a peripheral shape dimensioned to be at least as equal to a maximum outer dimension of void filling prosthesis 30 and preferably greater so that it seats against a resected surface of a tibia in use. For example, in the embodiment depicted, flange 66 is disc shaped, and void filling prosthesis 30 is frustoconical such that its maximum diameter is located at one end thereof. Flange 66 may have a diameter at least equal to the maximum diameter of prosthesis 30. However, flange 66 preferably has a diameter larger than that of prosthesis 30 so that it extends beyond the boundaries of void filling prosthesis 30 and substantially covers the resected surface or surfaces of a tibia.

Projection 68 on the other hand is dimensioned to substantially conform to an interior of void filling prosthesis 30. Thus, for the embodiment depicted, projection 68 matches the size and conical taper of interior cone surface 31 of void filling prosthesis 30. In this regard, projection 68 pressurizes the bone cement while flange 66 acts as a depth stop. Projection 68 extends distally from flange 66 and has a depression 63 extending proximally therein, as best shown in FIG. 6C. Projection 68 creates a relief space for bone cement to travel to while the bone cement within void filling prosthesis 30 is pressurized via projection 68. The snug fit of projection 68 and depression 63 helps ensure that bone cement does not escape from between projection 68 and interior cone surface 31. Since void filling prosthesis 30 may come in several different sizes, a kit may include several differently sized pressurizers 60 corresponding to each size void filling prosthesis 30.

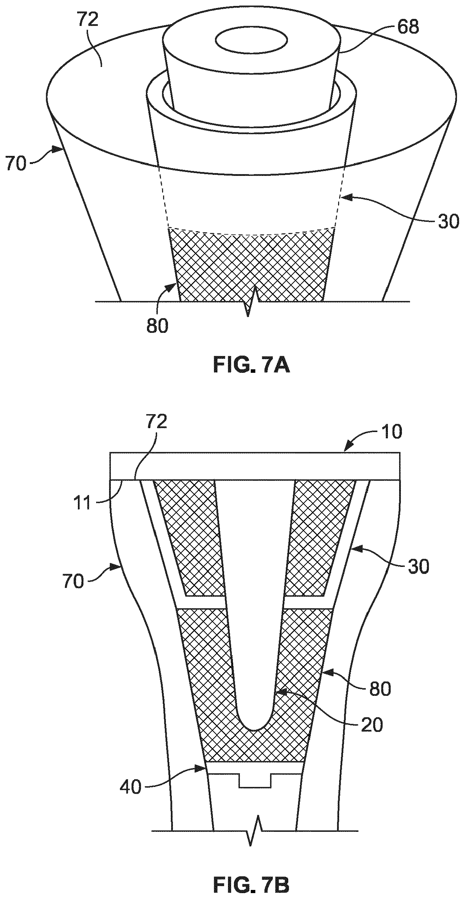

A method of using the foregoing instruments and implants to perform a revision TKA will now be described. In the revision procedure, a previously implanted prosthesis is removed from a tibia 70, and the proximal end of the tibia is resected to resurface the bone for the revision prosthesis. In other words, the proximal tibia is resected to form a proximal resected surface 72, as shown in FIG. 7A. Additionally, tibia 70 may be prepared for void filling prosthesis 30. Exemplary preparation techniques are described in the heretofore mentioned disclosures of U.S. Patent Nos. 9,011,444; 9,149,282; 10,524,806 and 10,299,929. Such preparation generally involves the resection of bone within a metaphysis of tibia 70 so that it is properly shaped to accommodate void filling prosthesis 30.

Once the proximal end of tibia 70 has been prepared, cement plug 40 is inserted into the intramedullary canal of tibia 70 so that it is positioned within the diaphysis of bone 70 distal of the metaphysis. Thereafter, void filling prosthesis 30 is implanted into tibia 70 preferably in a press-fit manner so that its porous exterior surface 32 bears against the bleeding cancellous bone of the metaphysis. Bone cement 80 is then injected or otherwise inserted into tibia 70 so that the space between cement plug 40 and resected surface 72, including channel 34 of void filling prosthesis 30, is substantially filled with bone cement 80. Bone cement 80 is then pressurized by inserting projection 68 of pressurizer 60 into channel 34 of void filling prosthesis 30 and either pushing down on the bone cement 80 by hand or using a mallet to impact an impaction tool connected to pressurizer 60 to tamp bone cement 80 to the desired pressure, as shown in FIG. 7A. At this point, resected surface 72 should be substantially free of bone cement 80.

Cement shield 50 is then placed onto resected surface 72. In this regard, shield bodies 52 are moved to the closed position and placed into contact with resected surface 72 so that channel 58 is centered over cement 80. While maintaining shield 50 against resected surface 72, stem 20 is inserted into cement 80 which causes cement 80 to flow or otherwise eject from tibia 70. Shield 50 prevents the overflow of cement 80 from coming into contact with resected surface 72 and instead directs the overflow up through channel 58 of shield 50 and around stem 20. The overflow cement is removed as it is expelled from bone 70. Just as baseplate 10 comes into contact or nearly comes into contact with shield bodies 52, shield 50 is removed from bone 70 by actuating handles 56 and moving shield bodies 52 to the open position. Tibial baseplate 10 is then seated onto the proximal tibia 70 so that porous distal surface 18 directly contacts resected surface 72 which should be substantially free of cement 80. Bone cement 80 is then allowed to cure. Thus, the interface between plate portion 12 and proximal tibia 72 is cementless, while the interface between stem 20 and bone 70/void filler 30 is cemented. Over time and with use, stem 20 may subside further within bone cement 80 which helps facilitate load transfer to the interface between plate portion 12 and resected surface 72. However, stem 20 remains cemented and helps resist rotational forces at least due to its cruciform shape.

It should be noted that stem 20 extends through channel 34 of void filling prosthesis 30 so that distal tip 27 of stem 20 is positioned within the diaphysis of tibia 70. In this regard, stem 20 has a length that facilitates the positioning of distal tip 27 within the diaphysis. However, stem 20 may have shortened length such that distal tip 27 terminates within the metaphysis of tibia 70. Since the metaphysis is generally less constraining than the diaphysis, such shortened stem 20 may allow the positioning of tibial baseplate 10 at different varus-valgus angles than just a neutral position. Additionally, the need for a stem offset may be precluded as the shortened stem 20 may allow baseplate 10 to be shifted medially-laterally and/or anteriorly-posteriorly to find the proper positioning of plate portion 12 on bone 70 prior to cement 80 curing. Additionally, some embodiments of the method may not utilize void filling prosthesis 30.

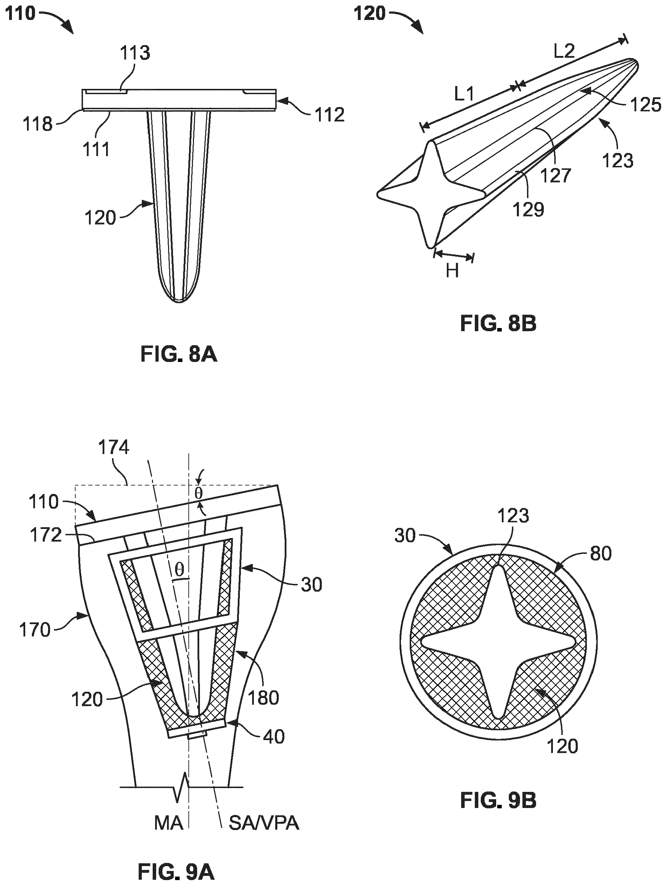

FIGs. 8A-8B depict a tibial baseplate 110 according to another embodiment of the present disclosure. For ease of review, like elements will be accorded like reference numerals to that of baseplate 10 and stem 20 but within the 100-series of numbers. Tibial baseplate 110 is a monolithic baseplate in that stem 120 is integral with plate portion 112. Plate portion 112 is similar to plate portion 12 in that it includes an articular side that defines a tray 113 for a tibial insert and an articular side that includes a layer of porous material 118 which defines a bone contact surface 111 configured for bony ingrowth.

Integral stem 120 is similar to stem 20 in that it includes a plurality of flutes 123 and grooves 125 that form a cruciform cross-section. However, flutes 123 of stem 120 have a dual axial taper. In this regard, flutes 123 each have a flute height H that extends from a root 127 to a tip 129 thereof. This flute height H decreases at two different rates, so that the distance between root 127 and tip 129 of each flute 123 decreases along a first length portion L1 of stem 120 at a first taper angle and a second length portion L2 of stem 120 at a second taper angle. The first taper angle may have the same taper angle as that of an interior of void filling prosthesis 30. The second taper angle may be a greater taper angle than that of the first taper angle. In other words, second length portion L2 may taper at a greater rate than L1 to facilitate subsidence. The transition between L1 and L2 may be dependent on a length of void filling prosthesis 30, for example. In this regard, L1 may be the same length as void filling prosthesis 30. In such embodiment, the transition between L1 and L2 may then be located a distance from a distal tip of stem 120 that is equal to the difference between the total length of stem 120 and L1 (or length of prosthesis 30). Such dual taper facilitates subsidence of stem 120 in bone cement and helps convert tension at the cement interface into compression. Moreover, subsidence, as mentioned above, allows for the reduction in any distance between a bone and bone contact surface 111 of baseplate 110 and facilitates complete and even loading perpendicular to this interface.

The first taper of stem 120 along first length portion L1 may match the taper of channel 30 within void filling prosthesis 30. Additionally, an outer periphery or dimension of stem 120 along length L1 may closely correspond to an inner dimension of void filling prosthesis 30 along channel 34. For example, stem flutes 123 and an inner surface 31 of void filling prosthesis 30 may have a maximum offset of about 1 cm. This allows stem 120 to dock to void filling prosthesis 30 while allowing stem 120 the ability to subside within bone cement between grooves 125 and void filling prosthesis 30. In this regard, void filling prosthesis 30 can help direct the orientation of baseplate portion 112 or whatever joint prosthesis is connected to stem 120.

FIGs. 9A and 9B illustrate a benefit of the docking effect as it can facilitate the use of a kinematic alignment revision procedure. As shown in the cross-section of FIG. 9B, stem flutes 123 and void filling prosthesis 30 in one cross-sectional plane have a total offset which is great enough to allow subsidence to proceed unimpeded but is constraining enough to restrict the angulation of a longitudinal axis SA of stem 120 relative to a longitudinal axis VPA of void filling prosthesis 30. In other words, stem 120 and void filling prosthesis 30 are self-aligning when stem 120 is docked to void filling prosthesis 30. This allows baseplate 110 and void filing prosthesis 30 to be used in a kinematic alignment revision procedure. As shown in FIG. 9A, a typical anatomic alignment procedure would resect tibia 170 parallel to a neutral joint line 174 so that resected surface 172 of tibia 170 is oriented perpendicular to a tibial mechanical axis MA. However, the patient's natural j oint line may be naturally varus or valgus. In this regard, a kinematic procedure may be performed so that the joint line, and correspondingly resected surface 172, is oriented at an angle Θ relative to the neutral joint line 174 and parallel to the patient's natural kinematic joint line. Longitudinal axes SA and VPA of void filling prosthesis 30 and stem 120, respectively, are similarly rotated relative to the tibial mechanical axis MA by angle Θ. In the procedure, a robot or surgeon with the aid of a computer-assisted haptic feedback system can be used to shape bone 170 for void filling prosthesis 30 and tibial component 110. Exemplary manual and computer-assisted kinematic alignment techniques that may be used to resect the bones of the knee joint are respectively disclosed in U.S. Patent No. 8,974,459 and U.S. Pub. No. 2021/0346036. In this regard, a conical burr or reamer may be used form resected surface 172 to shape the metaphyseal bone cavity to receive void filling prosthesis 30 at the desired orientation. Once void filling prosthesis 30 has been implanted and cement 180 inserted into the cavity between plug 40 and resected surface 172, stem 120 of baseplate 110 is then inserted into void filling prosthesis 30. The conformal nature of stem 120 and void filling prosthesis 30 self-orients stem 120 and, consequently, tibial baseplate 110 to the proper kinematic orientation. It is noted that shield 50 may also be used to prevent cement contamination of resected surface 172 so that direct contact can be made between porous material 118 of baseplate 110 and resected surface 172.

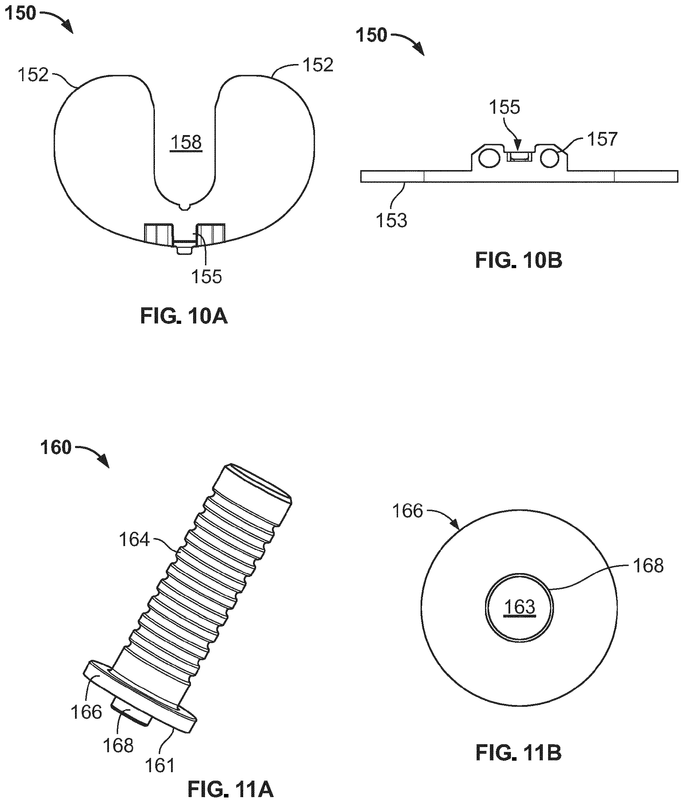

FIGs. 10A-10B depict a cement shield 150 according to another embodiment of the present disclosure. For ease of review, like elements will be accorded like reference numerals to that of cement shield 50 but within the 100-series of numbers. Shield 150 is similar to shield 50 in that it includes shield bodies 152 shaped to overlap a resected proximal tibia. In addition, a channel 158 is formed between shield bodies 152. However, unlike shield 50, bodies 152 are rigidly fixed relative to each other via a bridge 155 extending between them. Thus, channel 158 runs out a posterior side of shield 150 to facilitate its removal from a stem since channel 158 cannot be opened via actuation of a handle. In this regard, shield 150 does not have moveable handles. Instead, bridge 155 may have a tool interface 157 configured to receive a separate handle for manipulation of shield. Alternatively, an integral handle may be connected to shield 150 at this tool interface 157.

FIGs. 11A-11B depict a cement pressurizer 160 according to another embodiment of the present disclosure. For ease of review, like elements will be accorded like reference numerals to that of cement pressurizer 60 but within the 100-series of numbers. Cement pressurizer 160 is similar to pressurizer 60 in that it includes a shaft 164 with a flange or plate 166 disposed at the end of shaft 164 and a projection 168 extending from flange 166. Moreover, projection 168 is shaped and sized to be received within channel 34 of void filling prosthesis 30. However, pressurizer 160 is not configured to connect to another tool, and shaft 164 forms a handle for manual manipulation.

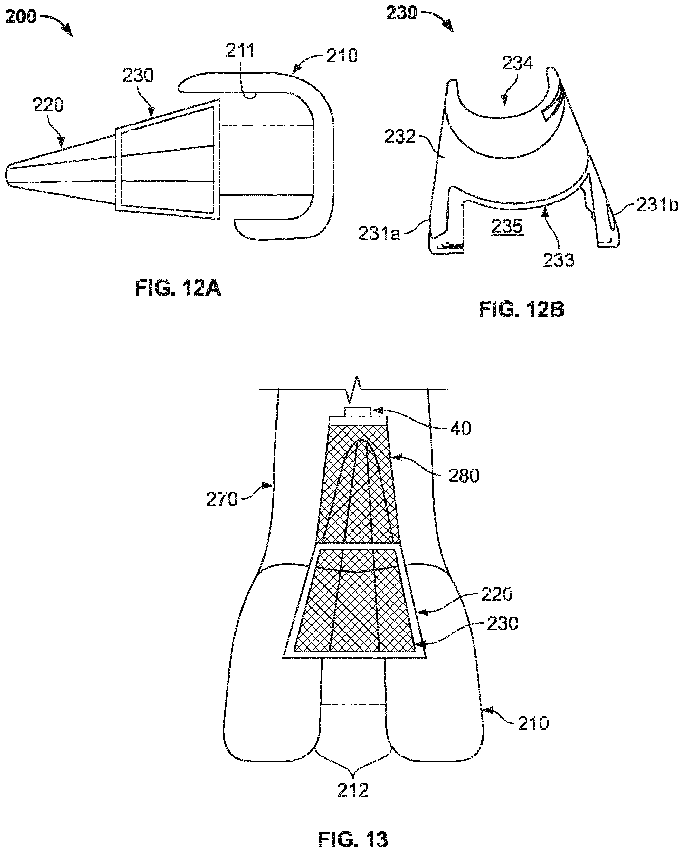

FIGs. 12A and 12B depicts a femoral assembly 200 according to an embodiment of the present disclosure. Femoral assembly 200 generally includes a femoral component 210, a stem 220 extending from femoral component 210, and a void filling prosthesis 230. Femoral component 210 includes an articular side and a bone contact side. The articular side has a pair of condyles 212 for articulation with corresponding condyles of a tibial insert. The bone contact side includes a plurality of intersecting planar surfaces, such as three to five planar surfaces, for example. One or more of such planar surfaces includes a layer of porous material 211 for bony ingrowth. Stem 220 extends from femoral component 210 and may be similar to either stem 30 or 130. However, in the embodiment depicted it is similar to stem 130 in that it has a periphery configured for docking with void filling prosthesis 230.

Void filling prosthesis 230, as shown in FIG. 12, has a frustoconical shape and includes a central body 233 and a pair of opposing legs 231a-b that define a space 235 therebetween. A channel 234 extends axially through central body 235 in a proximodistal direction and also through body 233 in an anterior direction which helps provide clearance space between void filling prosthesis 230 and femoral component 211. An exterior surface of void filling prosthesis 230 includes a layer of a porous material 232 for bony ingrowth. Other configurations for femoral void filling prosthesis are contemplated, such as those disclosed in the heretofore mentioned disclosures of U.S. Patent Nos. 9,011,444; 9,149,282; and 10,524,806.

FIG. 13 depicts a femoral assembly 200 implanted onto a distal femur 270. The method for implantation is the same as that described above with respect to the tibial devices except that the procedure is performed on a femur 270 using analog femoral devices. In this regard, femur 270 is prepared, bone plug 40 is inserted into femur 270 followed by void filling prosthesis 230, cement 280 is inserted, cement 280 is pressurized, a cement shield configured for femur 270 is applied one or more resected surfaces of bone 270 surrounding the cement filled void, and stem 220 is inserted into bone cement 280 while shielding the resected surfaces of femur 270 from bone cement 280.

A total knee arthroplasty system may include tibial baseplate 10 or 110, stem 20 or 120, void filling prosthesis 30 and 230, cement plug 40, cement shield 50 or 150, and cement pressurizer 60 and 160. In addition, system may include femoral assembly 200. A kit may include multiple sizes of each of such system devices.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

## Claims

1. A total knee arthroplasty system, comprising:
a joint prosthesis having a joint component (10, 210) and a stem (20) configured to extend from the joint component, the joint component having an articular side (13) and a bone contact side, the articular side having first and second condyles, and the bone contact side having a porous bone contact surface (18); and
a void filling prosthesis (30) having a porous outer surface (32) and a channel (34) extending entirely through the void filling prosthesis and configured to receive the stem of the joint prosthesis;
**characterised in that** the total knee arthroplasty system further comprises
a cement plug (40); and
a cement shield (50) having a first member (51a) and a second member (51b), the first member and the second member defining a channel (58) extending through the cement shield and having a planar surface (53) configured to abut a resected surface of a bone so that the channel aligns with an intramedullary canal of the bone.

2. The system of claim 1, wherein the first and second members of the shield are moveable relative to each other from an open position to a closed position.

3. The system of claim 2, wherein each of the first and second members each include a shield body (52) having a notch (54) such that when the first and second members are in the closed position, the notches of the first and second members come together to form the channel.

4. The system of claim 3, wherein the first and second members are pivotably connected to each other.

5. The system of claim 4, wherein the first and second members include a ratchet mechanism (57) that secures the first and second members while in the open and closed positions

6. The system of claim 1, further comprising a cement pressurizer (60) having an elongate shaft (64) and a flange (66) extending radially outwardly from an end of the elongate shaft.

7. The system of claim 6, wherein the flange has a periphery corresponding to a periphery of the channel of the void filling prosthesis.

8. The system of claim 6, wherein the cement pressurizer includes a projection (68) extending from the flange in an axial direction and having a depression (63) therein.

9. The system of claim 8, wherein the projection is cylindrical.

10. The system of claim 1, wherein the joint prosthesis is a tibial prosthesis, and the joint component is a tibial baseplate (10).

11. The system of claim 10, wherein the void filling prosthesis includes notches (36) extending through a sidewall thereof and configured to receive keels (16) of the tibial baseplate

12. The system of claim 10, wherein the stem includes a connection portion (21) and a stem portion (22), the stem portion includes a plurality of flutes (23) and grooves forming a cruciform cross-sectional shape.

13. The system of claim 12, wherein each flute is tapered in a proximodistal direction and a direction transverse to the proximodistal direction.

14. The system of claim 1, wherein the joint prosthesis is a femoral prosthesis, and the joint component is a distal femoral component (210)

15. The system of claim 14, wherein void filling prosthesis (230) has a frustoconical shape and includes a central body (233) and a pair of opposing legs (231a-b) that define a space therebetween.

## Patentansprüche

1. Gesamtkniearthroplastiksystem, das Folgendes umfasst:
eine Gelenkprothese mit einer Gelenkkomponente (10, 210) und einem Schaft (20), der dazu ausgelegt ist, sich von der Gelenkkomponente zu erstrecken, wobei die Gelenkkomponente eine artikuläre Seite (13) und eine Knochenkontaktseite aufweist, wobei die artikuläre Seite einen ersten und einen zweiten Kondylus aufweist, und wobei die Knochenkontaktseite eine poröse Knochenkontaktfläche (18) aufweist; und
eine Hohlraumfüllprothese (30) mit einer porösen Außenfläche (32) und einer Durchführung(34), die sich vollständig durch die Hohlraumfüllprothese erstreckt und dazu ausgelegt ist, den Schaft der Gelenkprothese aufzunehmen;
**dadurch gekennzeichnet, dass** das Gesamtkniearthroplastiksystem ferner Folgendes umfasst
einen Zementstopfen (40); und
einen Zementschild (50) mit einem ersten Element (51a) und einem zweiten Element (51b), wobei das erste Element und das zweite Element eine Durchführung (58) definieren, die sich durch den Zementschild erstreckt und eine planare Fläche (53) aufweist, die dazu ausgelegt ist, an einer herausgeschnittenen Fläche eines Knochens anzuliegen, derart, dass die Durchführung auf einen intramedullären Kanal des Knochens ausgerichtet ist.

2. System nach Anspruch 1, wobei das erste und das zweite Element des Schildes relativ zueinander aus einer geöffneten Position in eine geschlossene Position bewegbar sind.

3. System nach Anspruch 2, wobei jedes des ersten und des zweiten Elements jeweils einen Schildkörper (52) beinhalten, der eine Kerbe (54) aufweist, derart, dass, wenn sich das erste und das zweite Element in der geschlossenen Position befinden, die Kerben des ersten und des zweiten Elements zusammenkommen, um die Durchführung zu bilden.

4. System nach Anspruch 3, wobei das erste und das zweite Element schwenkbar miteinander verbunden sind.

5. System nach Anspruch 4, wobei das erste und das zweite Element einen Ratschenmechanismus (57) beinhalten, der das erste und das zweite Element sichert, wenn sie sich in der geöffneten und der geschlossenen Position befinden.

6. System nach Anspruch 1, das ferner einen Zementdruckhalter (60) mit einer langgestreckten Welle (64) und einem Flansch (66), der sich von einem Ende der langgestreckten Welle radial nach außen erstreckt, aufweist.

7. System nach Anspruch 6, wobei der Flansch einen Umfang aufweist, der einem Umfang der Durchführung der Hohlraumfüllprothese entspricht.

8. System nach Anspruch 6, wobei der Zementdruckhalter einen Vorsprung (68) beinhaltet, der sich vom Flansch in einer Axialrichtung erstreckt und eine Vertiefung (63) darin aufweist.

9. System nach Anspruch 8, wobei der Vorsprung zylindrisch ist.

10. System nach Anspruch 1, wobei die Gelenkprothese eine tibiale Prothese ist, und die Gelenkkomponente eine tibiale Grundplatte (10) ist.

11. System nach Anspruch 10, wobei die Hohlraumfüllprothese Kerben (36) beinhaltet, die sich durch eine Seitenwand davon erstrecken und dazu ausgelegt sind, Kiele (16) der tibialen Grundplatte aufzunehmen.

12. System nach Anspruch 10, wobei der Schaft einen Verbindungsabschnitt (21) und einen Schaftabschnitt (22) beinhaltet, wobei der Schaftabschnitt eine Vielzahl von Rillen (23) und Nuten beinhaltet, die eine kreuzförmige Querschnittsform bilden.

13. System nach Anspruch 12, wobei jede Rille in einer proximodistalen Richtung und einer Richtung quer zur proximodistalen Richtung konisch ist.

14. System nach Anspruch 1, wobei die Gelenkprothese eine femorale Prothese ist und die Gelenkkomponente eine distale femorale Komponente (210) ist.

15. System nach Anspruch 14, wobei die Hohlraumfüllprothese (230) eine Kegelstumpfform aufweist und einen zentralen Körper (233) und ein Paar von gegenüberliegenden Beinen (231a-b), die einen Raum dazwischen definieren, beinhaltet.

## Revendications

1. Système d'arthroplastie totale du genou, comprenant :
une prothèse articulaire comprenant un composant articulaire (10, 210) et une tige (20) configurée pour s'étendre à partir du composant articulaire, le composant articulaire ayant une face articulaire (13) et une face de contact d'os, la face articulaire ayant un premier et un second condyles, et la face de contact d'os ayant une surface de contact d'os poreuse (18) ; et
une prothèse de remplissage de vide (30) ayant une surface extérieure poreuse (32) et un canal (34) s'étendant entièrement à travers la prothèse de remplissage de vide et configuré pour recevoir la tige de la prothèse articulaire ;
**caractérisé par le fait que** le système d'arthroplastie totale du genou comprend en outre
un bouchon de ciment (40) ; et
une plaque de ciment (50) ayant un premier élément (51a) et un second élément (51b), le premier élément et le second élément définissant un canal (58) s'étendant à travers la plaque de ciment et ayant une surface plane (53) configurée pour venir en buter sur une surface réséquée d'un os de sorte que le canal s'aligne sur un canal intramédullaire de l'os.

2. Système selon la revendication 1, dans lequel le premier et le second élément de la plaque de ciment sont mobiles l'un par rapport à l'autre, d'une position ouverte à une position fermée.

3. Système selon la revendication 2, dans lequel le premier et le deuxième élément comprennent chacun un corps de plaque (52) comportant une encoche (54) de sorte que lorsque le premier et le deuxième élément sont en position fermée, les encoches du premier et du deuxième élément se rejoignent pour former le canal.

4. Système selon la revendication 3, dans lequel le premier et le second élément sont connectés de manière pivotante l'un à l'autre.

5. Système selon la revendication 4, dans lequel le premier et le deuxième élément comprennent un mécanisme à cliquet (57) qui maintien le premier et le deuxième élément en position ouverte et fermée.

6. Système selon la revendication 1, comprenant en outre un pressuriseur de ciment (60) ayant un arbre allongé (64) et une bride (66) s'étendant radialement vers l'extérieur à partir d'une extrémité de l'arbre allongé.

7. Système selon la revendication 6, dans lequel la bride a une périphérie correspondant à une périphérie du canal de la prothèse de remplissage de vide.

8. Système selon la revendication 6, dans lequel le pressuriseur de ciment comprend une projection (68) s'étendant à partir de la bride dans une direction axiale et formant une dépression (63).

9. Système selon la revendication 8, dans lequel la projection est cylindrique.

10. Système selon la revendication 1, dans lequel la prothèse articulaire est une prothèse tibiale, et le composant articulaire est une plaque de base tibiale (10).

11. Système selon la revendication 10, dans lequel la prothèse de remplissage de vide comprend des encoches (36) s'étendant à travers une de ses parois latérales et configurées pour recevoir des quilles (16) de la plaque de base tibiale.

12. Système selon la revendication 10, dans lequel la tige comprend une portion de connexion (21) et une portion de tige (22), la portion de tige comprenant une pluralité de cannelures (23) et de rainures formant une section transversale cruciforme.

13. Système selon la revendication 12, dans lequel chaque cannelure est conique dans une direction proximodistale et dans une direction transversale à la direction proximodistale.

14. Système selon la revendication 1, dans lequel la prothèse articulaire est une prothèse fémorale, et le composant articulaire est un composant fémoral distal (210).

15. Système selon la revendication 14, dans lequel la prothèse de remplissage de vide (230) a une forme tronconique et comprend un corps central (233) et une paire de jambes opposées (231a-b) qui définissent un espace entre elles.
